# EUROPEAN PATENT APPLICATION

(11) **EP 4 717 150 A1**
(43) Date of publication of application: **01.04.2026**
(21) Application number: 25202694.3
(22) Date of filing: 17.09.2025
(51) Int. Cl.: A61B 3/10, A61B 3/12

(54) **SCANNING LASER OPHTHALMOSCOPE CAPABLE OF OBTAINING FLUORESCENT IMAGES OF OCULAR FUNDUS**

(30) Priority: 25.09.2024 KR 20240129861
(71) Applicant: Huvitz Co., Ltd., Anyang-si, Gyeonggi-do 14055 (KR)
(72) Inventor: JO, Sun Hyung, 14055 Anyang-si (KR); BEAK, Woo Kyung, 14055 Anyang-si (KR)
(74) Representative: Henkel & Partner mbB

(57) **Abstract**

Disclosed is a scanning laser ophthalmoscope capable of obtaining visible light images and fluorescent images of the ocular fundus. The scanning laser ophthalmoscope includes a visible light source (30); an infrared light source (32); a scanner (40) configured to irradiate measurement lights onto a desired location on the ocular fundus of an eye to be examined (5) in sequence; a beam splitter (44) configured to separate the paths of the measurement lights and the reflected light; and a light detection unit (50) for detecting the reflected light, wherein the light detection unit (50) includes a red light separation mirror (52a) configured to separate red light with a wavelength of 600 to 800 nm from the reflected light; a red light detection unit (52c) configured to detect the red light separated by the red light separation mirror (52a) and generate a red image of the ocular fundus; a green light separation mirror (54a) configured to separate green light with a wavelength of 500 to 550 nm from the reflected light; a green light detection unit (54c) configured to detect the green light separated by the green light separation mirror (54a) and generate a green image of the ocular fundus; a variable filter (60) configured to selectively transmit blue light with a wavelength of 400 to less than 500 nm or infrared light with a wavelength of greater than 800 nm to 900 nm from the reflected light; and a blue light and fluorescence detection unit (56c) configured to detect the blue light with a wavelength of 400 to less than 500 nm and the infrared light with a wavelength of greater than 800 nm to 900 nm that have passed through the variable filter (60) and generate a blue image and an infrared image of the ocular fundus.

## Description

### CROSS-REFERENCE TO RELATED APPLICATION

This application claims benefit of priority to Korean Patent Application No. 10-2024-0129861 filed on September 25, 2024, the entire contents of which are incorporated herein by reference.

### FIELD OF THE DISCLOSURE

The present disclosure relates to a scanning laser ophthalmoscope, and more particularly, to a scanning laser ophthalmoscope capable of obtaining visible light images and fluorescent images of the ocular fundus.

### BACKGROUND

A scanning laser ophthalmoscope (SLO) is an examination device that projects laser light emitted from a light source onto the ocular fundus of an examinee and obtains an image of the ocular fundus by detecting the reflected light reflected off the ocular fundus. Ocular fundus examinations are used not only in ophthalmology but also in internal medicine for the diagnosis of hypertension, diabetes, neurological disorders, and the like.

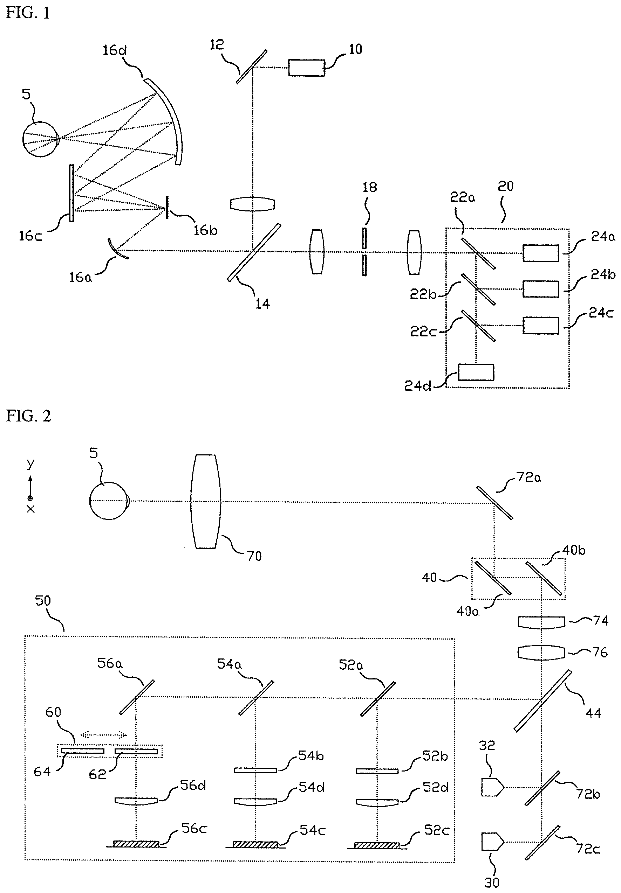

FIG. 1 is a diagram showing the configuration of a typical scanning laser ophthalmoscope. As shown in FIG. 1, in the typical scanning laser ophthalmoscope (SLO), a measurement light, specifically, laser light of a red (R), green (G), blue (B), or infrared (IR) wavelength, is emitted from a light source 10, and the emitted measurement light is reflected by a reflective mirror 12, a beam splitter 14, flat or concave reflective mirrors 16a, 16b, 16c, and 16d, etc., and is incident on the ocular fundus of the eye to be examined 5. At this time, some of the reflective mirrors 16b are driven by a galvanometer to change the reflection angle of the measurement light, thereby allowing the measurement light to be incident on the desired measurement location of the ocular fundus.

The reflected light reflected off the ocular fundus of the eye to be examined 5 travels in the opposite direction to the measurement light, is reflected by the reflective mirrors 16a, 16b, 16c, and 16d, passes through the beam splitter 14 and the confocal slit 18, and is then detected by the light detection unit 20. The confocal slit 18 serves to eliminate unnecessary scattered light of the eye to be examined 5 and the optical system. The reflected light incident on the light detection unit 20 is reflected by or passes through two or more blocking filters 22a, 22b, and 22c according to the wavelength of the reflected light, is separated by wavelength, and is detected respectively by wavelength-specific detectors 24a, 24b, 24c, and 24d. For example, the first blocking filter 22a passes red (R) reflected light and reflects the rest of the reflected light, the second blocking filter 22b reflects green (G) reflected light and passes the rest of the reflected light, and the third blocking filter 22c reflects blue (B) reflected light and passes the rest of the reflected light. The rest of the reflected light that has passed through the third blocking filter 22c, for example, infrared (IR) reflected light, is detected by the infrared detector 24d.

As such, in the scanning laser ophthalmoscope (SLO), a white measurement light must be realized using three primary color lasers of red (R), green (G), and blue (B) in order to obtain an image of the ocular fundus illuminated with white light, and the detectors 24a, 24b, and 24c for detecting red (R), green (G), and blue (B) must be used in order to reimage the reflected light of each color. Further, the separate infrared detector 24d must be used in order to obtain an infrared image of the ocular fundus.

In other words, in the conventional scanning laser ophthalmoscope (SLO), the respective filters 22a, 22b, and 22c and wavelength-specific detectors 24a, 24b, 24c, and 24d must be used in order to separate and detect the reflected light reflected off the ocular fundus according to its wavelength. However, if a number of filters 22a, 22b, and 22c and wavelength-specific detectors 24a, 24b, 24c, and 24d are used in this way, not only does the device structure of the scanning laser ophthalmoscope become complicated and inefficient, but there is also a problem that it is difficult to obtain ocular fundus images of various wavelengths, such as fluorescent images of the ocular fundus.

### Prior Art Literature

Japanese Patent Application Publication No. 2016-123467 (Publication date: July 11, 2016)

### SUMMARY OF THE DISCLOSURE

### TECHNICAL OBJECTS

It is an object of the present disclosure to provide a scanning laser ophthalmoscope capable of obtaining not only visible light images of the ocular fundus but also ocular fundus images of various wavelengths, such as fluorescent images of the ocular fundus.

It is another object of the present disclosure to provide a scanning laser ophthalmoscope capable of detecting ocular fundus images of two or more wavelengths by using a single detector.

### TECHNICAL SOLUTION

In order to achieve the above objects, the present disclosure provides a scanning laser ophthalmoscope including a visible light source 30 configured to emit a visible measurement light; an infrared light source 32 configured to emit an infrared measurement light; a scanner 40 configured to adjust reflection angles of the visible measurement light and the infrared measurement light and irradiate the measurement lights onto a desired location on an ocular fundus of an eye to be examined 5 in sequence; a beam splitter 44 configured to change a path of reflected light formed by the measurement lights being reflected off the ocular fundus of the eye to be examined 5 and separate paths of the measurement lights and the reflected light; and a light detection unit 50 for detecting the reflected light,

wherein the light detection unit 50 includes a red light separation mirror 52a configured to separate red light with a wavelength of 600 to 800 nm from the reflected light; a red light detection unit 52c configured to detect the red light separated by the red light separation mirror 52a and generate a red image of the ocular fundus; a green light separation mirror 54a configured to separate green light with a wavelength of 500 to 550 nm from the reflected light; a green light detection unit 54c configured to detect the green light separated by the green light separation mirror 54a and generate a green image of the ocular fundus; a variable filter 60 configured to selectively transmit blue light with a wavelength of 400 to less than 500 nm or infrared light with a wavelength of greater than 800 nm to 900 nm from the reflected light; and a blue light and fluorescence detection unit 56c configured to detect the blue light with a wavelength of 400 to less than 500 nm and the infrared light with a wavelength of greater than 800 nm to 900 nm that have passed through the variable filter 60 and generate a blue image and an infrared image of the ocular fundus.

### EFFECTS OF THE DISCLOSURE

According to the scanning laser ophthalmoscope in accordance with the present disclosure, not only visible light images of the ocular fundus but also ocular fundus images of various wavelengths, such as fluorescent images of the ocular fundus, can be obtained. Further, according to the scanning laser ophthalmoscope in accordance with the present disclosure, ocular fundus images of two or more wavelengths can be detected using a single detector.

### BRIEF DESCRIPTION OF THE DRAWINGS

FIG. 1 is a diagram showing the configuration of a typical scanning laser ophthalmoscope;
FIG. 2 is a diagram showing the structure of a scanning laser ophthalmoscope according to one embodiment of the present disclosure; and
FIGS. 3 and 4 are diagrams for describing the structure and operating state of a variable filter 60 that can be used in a scanning laser ophthalmoscope according to one embodiment of the present disclosure.

### DETAILED DESCRIPTION OF THE PREFERRED EMBODIMENTS

Hereinafter, the present disclosure will be described in detail with reference to the accompanying drawings.

FIG. 2 is a diagram showing the structure of a scanning laser ophthalmoscope according to one embodiment of the present disclosure. As shown in FIG. 2, the scanning laser ophthalmoscope according to the present disclosure includes a visible light source 30 that emits a visible measurement light, an infrared light source 32 that emits an infrared measurement light, a scanner 40 that adjusts the reflection angles of the visible measurement light and the infrared measurement light and sequentially irradiates the measurement lights onto a desired location on the ocular fundus of the eye to be examined 5, a beam splitter 44 that changes the path of the reflected light formed by the measurement lights being reflected off the ocular fundus of the eye to be examined 5 and separates the paths of the measurement lights and the reflected light, and a light detection unit 50 for detecting the reflected light.

The visible light source 30 emits a measurement light of a visible light wavelength in order to obtain a visible light image of the ocular fundus, and generates, for example, a monochromatic laser visible light, specifically, a red (R), green (G), or blue (B) laser measurement light. The infrared light source 32 emits an infrared (IR) measurement light in order to obtain an infrared or fluorescent image of the ocular fundus.

The scanner 40 irradiates the measurement lights onto the ocular fundus of the eye to be examined 5 through the pupil of the eye to be examined 5, and allows the measurement lights to be irradiated in sequence onto each location on the surface (x-y plane) of the ocular fundus. For example, the scanner 40 may include an x-direction scanner 40a that changes the x-direction irradiation location of the measurement lights and a y-direction scanner 40b that changes the y-direction irradiation location of the measurement lights, and may allow the measurement lights to be irradiated in sequence onto each location on the ocular fundus by changing the locations of the x-direction scanner 40a and the y-direction scanner 40b in sequence. In other words, the measurement lights are sequentially scanned over the surface of the ocular fundus of the eye to be examined 5 by the scanner 40.

The beam splitter 44 separates the paths of the measurement lights and of the reflected light formed by the measurement lights being reflected off the ocular fundus of the eye to be examined 5, and guides the reflected light to the light detection unit 50. The beam splitter 44 may be a typical device that passes the measurement lights and reflects the reflected light, and, for example, may be a hole mirror in which a hole 14a for passing the measurement lights is formed in the center and a mirror for reflecting the reflected light is formed around the hole 14a, as shown in FIG. 1.

The light detection unit 50 includes a red light separation mirror 52a, a red light detection unit 52c, a green light separation mirror 54a, a green light detection unit 54c, a variable filter 60, and a blue light and fluorescence detection unit 56c.

The red light separation mirror 52a separates red light with a wavelength of 600 to 800 nm from the reflected light, and specifically, separates red light with a wavelength of 600 to 800 nm from the reflected light with a wavelength of 400 to 550 nm and greater than 800 nm to 900 nm. For example, the red light separation mirror 52a may reflect red light with a wavelength of 600 to 800 nm and transmit the reflected light with a wavelength of 400 to 550 nm and greater than 800 nm to 900 nm. The red light separated by the red light separation mirror 52a is detected by the red light detection unit 52c, and a red image of the ocular fundus is generated. At this time, as needed, the red light separated by the red light separation mirror 52a may further pass through a red light filter 52b that transmits red light with a wavelength of 600 to 800 nm and blocks the rest of the reflected light.

The red light separation mirror 52a separates red light with a wavelength of 600 to 800 nm from the reflected light, and can thus separate infrared light with a wavelength of 750 to 800 nm from the reflected light. Therefore, if an infrared measurement light is irradiated onto the ocular fundus by using the infrared light source 32, infrared reflected light with a wavelength of 750 to 800 nm reflected off the ocular fundus can be detected using the red light detection unit 52c, and an infrared image of the ocular fundus can be obtained.

The green light separation mirror 54a separates green light with a wavelength of 500 to 550 nm from the reflected light, and specifically, separates green light with a wavelength of 500 to 550 nm from the reflected light with a wavelength of 400 to less than 500 nm and 800 to 900 nm. For example, the green light separation mirror 54a may reflect green light with a wavelength of 500 to 550 nm and transmit the reflected light with a wavelength of 400 to less than 500 nm and 800 to 900 nm. The green light separated by the green light separation mirror 54a is detected by the green light detection unit 54c, and a green image of the ocular fundus is generated. At this time, as needed, the green light separated by the green light separation mirror 54a may further pass through a green light filter 54b that transmits green light with a wavelength of 500 to 550 nm and blocks the rest of the reflected light.

The green light separation mirror 54a separates green light with a wavelength of 500 to 550 nm from the reflected light, and can thus generate not only a green image of the ocular fundus itself but also a fluorescein angiography (FA) image or an autofluorescence (AF) image of the ocular fundus if a fluorescent substance such as fluorescein is injected into the blood vessels of the eye to be examined 5.

The variable filter 60 selectively transmits blue light with a wavelength of 400 to less than 500 nm or infrared light with a wavelength of greater than 800 nm to 900 nm from the reflected light. The blue light with a wavelength of 400 to less than 500 nm and the infrared light with a wavelength of greater than 800 nm to 900 nm that have passed through the variable filter 60 are detected by the blue light and fluorescence detection unit 56c.

The blue light and fluorescence detection unit 56c detects blue light and generates a blue image of the ocular fundus. Further, if a fluorescent substance such as indocyanine green (ICG) is injected into the blood vessels of the eye to be examined 5 and an infrared measurement light is irradiated onto the ocular fundus by using the infrared light source 32, fluorescent reflected light having a wavelength of 830 nm or higher, i.e., infrared reflected light, is generated from the ocular fundus of the eye to be examined 5. The blue light and fluorescence detection unit 56c detects the infrared reflected light having a wavelength of 830 nm or higher and generates an indocyanine green angiography (ICGA) fluorescent image of the ocular fundus. In other words, the blue light and fluorescence detection unit 56c can generate not only a blue image but also an infrared image, specifically, an indocyanine green angiography fluorescent image having a wavelength of 830 nm or higher, of the ocular fundus.

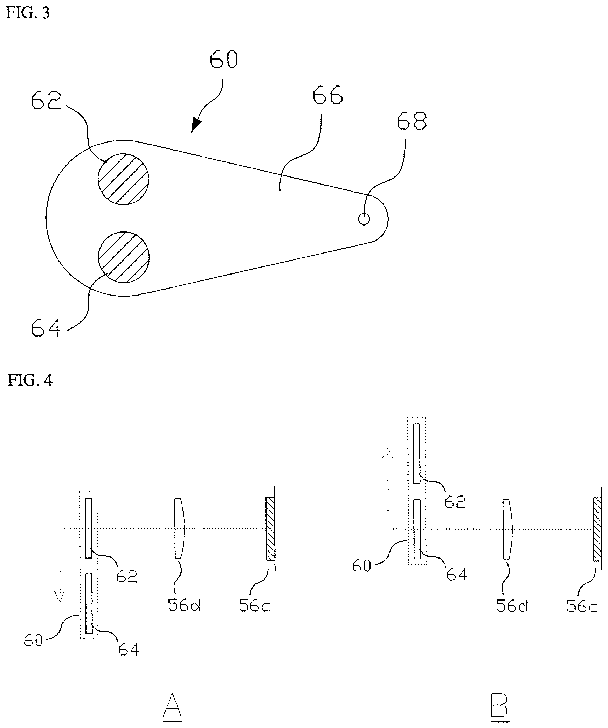

FIGS. 3 and 4 are diagrams for describing the structure and operating state of a variable filter 60 that can be used in a scanning laser ophthalmoscope according to one embodiment of the present disclosure.

As shown in FIG. 3, the variable filter 60 that can be used in the present disclosure includes a blue light filter 62 that separates blue light with a wavelength of 400 to less than 500 nm from the reflected light and an infrared filter 64 that separates infrared light with a wavelength of greater than 800 nm to 900 nm from the reflected light, and the blue light filter 62 and the infrared filter 64 are selectively positioned in the travel path of the reflected light. For example, the blue light filter 62 may transmit blue light with a wavelength of 400 to less than 500 nm and block the reflected light of all other wavelengths. The infrared filter 64 may transmit infrared light with a wavelength of greater than 800 nm to 900 nm and block the reflected light of 400 to 800 nm, and may be used as an ICGA filter for obtaining an indocyanine green angiography (ICGA) fluorescent image.

As shown in FIG. 3, the variable filter 60 may have a structure in which the blue light filter 62 and the infrared filter 64 are mounted on one end of a rotating plate 66 that rotates around a rotation shaft 68. When the rotating plate 66 rotates counterclockwise around the rotation shaft 68, the blue light filter 62 of the variable filter 60 is positioned in the travel direction of the reflected light (see A in FIG. 4), and the blue light with a wavelength of 400 to less than 500 nm is detected by the blue light and fluorescence detection unit 56c. On the other hand, when the rotating plate 66 rotates clockwise around the rotation shaft 68, the infrared filter 64 of the variable filter 60 is positioned in the travel direction of the reflected light (see B in FIG. 4), and the infrared light with a wavelength of greater than 800 nm to 900 nm is detected by the blue light and fluorescence detection unit 56c. In other words, the blue light filter 62 and the infrared filter 64 are used alternatively depending on the measurement image (visible light or infrared image).

The scanning laser ophthalmoscope according to one embodiment of the present disclosure may further include typical optical elements for guiding the measurement lights to the ocular fundus of the eye to be examined 5 and guiding the reflected light to the light detection unit 50, specifically, the red light detection unit 52c, the green light detection unit 54c, and the blue light and fluorescence detection unit 56c.

Specifically, as shown in FIG. 2, the scanning laser ophthalmoscope according to the present disclosure may further include an objective lens 70 that focuses the measurement light so that the focal point of the measurement light is formed on the ocular fundus of the eye to be examined 5 and focuses the reflected light reflected off the ocular fundus, one or more reflective mirrors 72a, 72b, 72c, and 56c that appropriately change the travel path of the measurement light or reflected light so that the devices constituting the optical system are appropriately arranged, a relay lens 74 that transmits the measurement light or the reflected light, a diopter lens 76 that changes the focal length of the measurement light according to the refractive power of the eye to be examined 5 so that the focal point of the measurement light is formed on the ocular fundus of the eye to be examined 5, focus lenses 52d, 54d, and 56d that focus the reflected light so that the focal points are formed on the red light detection unit 52c, the green light detection unit 54c, and the blue light and fluorescence detection unit 56c, confocal slits (18 in FIG. 1, not shown in FIG. 2) that are formed at the front end of the red light detection unit 52c, the green light detection unit 54c, and the blue light and fluorescence detection unit 56c and eliminate unnecessary scattered light of the eye to be examined 5 and the optical system, etc.

In the scanning laser ophthalmoscope according to the present disclosure, red, green, and blue measurement lights are respectively emitted by the visible light source 30, a red image, a green image, and a blue image of the ocular fundus of the eye to be examined 5 are respectively obtained using the red light detection unit 52c, the green light detection unit 54c, and the blue light and fluorescence detection unit 56c, and by integrating these, a color image of the ocular fundus can be obtained. Further, an infrared measurement light is emitted by the infrared light source 32, and an infrared image, a fluorescein angiography (FA) image or autofluorescence (AF) image, and an indocyanine green angiography fluorescent image having a wavelength of 830 nm or higher of the ocular fundus of the eye to be examined 5 can be obtained, respectively, using the red light detection unit 52c, the green light detection unit 54c, and the blue light and fluorescence detection unit 56c.

Although the present disclosure has been described above with reference to the accompanying drawings and example embodiments, the present disclosure is not limited to what is shown in the drawings and the embodiments described above but should be construed to encompass all modifications, and equivalent constructions and functions of the example embodiments within the scope set forth in the following claims. Further, reference numerals are indicated in the following claims to facilitate understanding, but the scope of the following claims is not limited to what is shown by the reference numerals and in the drawings.

## Claims

1. A scanning laser ophthalmoscope comprising:
a visible light source (30) configured to emit a visible measurement light;
an infrared light source (32) configured to emit an infrared measurement light;
a scanner (40) configured to adjust reflection angles of the visible measurement light and the infrared measurement light and irradiate the measurement lights onto a desired location on an ocular fundus of an eye to be examined (5) in sequence;
a beam splitter (44) configured to change a path of reflected light formed by the measurement lights being reflected off the ocular fundus of the eye to be examined (5) and separate paths of the measurement lights and the reflected light; and
a light detection unit (50) for detecting the reflected light,
wherein the light detection unit (50) comprises a red light separation mirror (52a) configured to separate red light with a wavelength of 600 to 800 nm from the reflected light; a red light detection unit (52c) configured to detect the red light separated by the red light separation mirror (52a) and generate a red image of the ocular fundus; a green light separation mirror (54a) configured to separate green light with a wavelength of 500 to 550 nm from the reflected light; a green light detection unit (54c) configured to detect the green light separated by the green light separation mirror (54a) and generate a green image of the ocular fundus; a variable filter (60) configured to selectively transmit blue light with a wavelength of 400 to less than 500 nm or infrared light with a wavelength of greater than 800 nm to 900 nm from the reflected light; and a blue light and fluorescence detection unit (56c) configured to detect the blue light with a wavelength of 400 to less than 500 nm and the infrared light with a wavelength of greater than 800 nm to 900 nm that have passed through the variable filter (60) and generate a blue image and an infrared image of the ocular fundus.

2. The scanning laser ophthalmoscope of claim 1, wherein the infrared light source (32) irradiates the ocular fundus with the infrared measurement light, and the blue light and fluorescence detection unit (56c) is configured to obtain an indocyanine green angiography (ICGA) fluorescent image by detecting infrared reflected light with a wavelength of 830 nm or higher.

3. The scanning laser ophthalmoscope of claim 1, wherein the infrared light source (32) irradiates the ocular fundus with the infrared measurement light, and the red light detection unit (52c) is configured to obtain an infrared image of the ocular fundus by detecting infrared reflected light with a wavelength of 750 to 800 nm reflected off the ocular fundus.

4. The scanning laser ophthalmoscope of claim 1, wherein the green light separation mirror (54a) generates a fluorescein angiography image or an autofluorescence image of the ocular fundus of the eye to be examined (5).

5. The scanning laser ophthalmoscope of claim 1, wherein the variable filter (60) comprises a blue light filter (62) configured to separate blue light with a wavelength of 400 to less than 500 nm from the reflected light and an infrared filter (64) configured to separate infrared light with a wavelength of greater than 800 nm to 900 nm from the reflected light, and the blue light filter (62) and the infrared filter (64) are selectively positioned in a path of the reflected light.

6. The scanning laser ophthalmoscope of claim 5, wherein the variable filter (60) has a structure in which the blue light filter (62) and the infrared filter (64) are mounted on one end of a rotating plate (66) that rotates around a rotation shaft (68).
